# EUROPEAN PATENT APPLICATION

(11) **EP 4 772 868 A1**
(43) Date of publication of application: **08.07.2026**
(21) Application number: 25211289.1
(22) Date of filing: 27.10.2025
(51) Int. Cl.: G01N 27/12

(54) **SEMICONDUCTOR DEVICE INCLUDING HYDROGEN SENSING STRUCTURE**

(30) Priority: 02.01.2025 KR 20250000548
(71) Applicant: SK hynix Inc., Bubal-eub Icheon-si Gyeonggi-do 17336 (KR)
(72) Inventor: CHA, Jun Hwe, 17336 Icheon-si (KR); KO, Dong Jin, 17336 Icheon-si (KR); KIM, Se Hyun, 17336 Icheon-si (KR); KIM, Wha Young, 17336 Icheon-si (KR); PARK, Gyeong Cheol, 17336 Icheon-si (KR)
(74) Representative: Isarpatent

(57) **Abstract**

A semiconductor device includes an integrated circuit structure disposed over a substrate and a hydrogen sensing structure disposed over the substrate to be laterally spaced apart from the integrated circuit structure. The integrated circuit structure includes an oxide semiconductor layer. The hydrogen sensing structure includes a hydrogen ion sensing layer containing a resistance change material, and a first sensing electrode layer and a second sensing electrode layer disposed at opposite ends of the hydrogen ion sensing layer. A bottom surface and a top surface of the hydrogen ion sensing layer are arranged to have same levels as a bottom surface and a top surface of the oxide semiconductor layer based on the surface of the substrate, respectively.

## Description

### CROSS-REFERENCE TO RELATED APPLICATIONS

The present application claims priority under 35 U.S.C §119(a) to Korean Application No. 10-2025-0000548, filed on January 2, 2025, the entire contents of which are incorporated herein by reference.

### BACKGROUND

### 1. Technical Field

Embodiments of the present disclosure generally relates to a semiconductor device, and more particularly, to a semiconductor device including a hydrogen sensing structure.

### 2. Related Art

Hydrogen ions can change electrical characteristics of a semiconductor device. Specifically, during semiconductor fabricating processes, hydrogen flowing into the semiconductor device from outside moves in the form of hydrogen ions inside the semiconductor device. The hydrogen ions may cause unintended changes in a surface potential or trap density of an active thin film in the semiconductor device, thereby deteriorating electrical characteristics of the active thin film. As a result, endurance or operational reliability of the semiconductor device may be reduced.

Hydrogen sensors for sensing hydrogen are often configured to sense hydrogen gas in the atmosphere by using a hydrogen sensing film exposed to an external air environment. In these instances, it may be difficult to directly apply the hydrogen sensor to a task of sensing hydrogen existing in an ionic state inside a semiconductor device. Accordingly, research is being conducted on devices that can effectively sense hydrogen in an ionic state inside the semiconductor device.

### SUMMARY

A semiconductor device according to an embodiment includes an integrated circuit structure disposed over a substrate and a hydrogen sensing structure disposed over the substrate to be laterally spaced apart from the integrated circuit structure. The integrated circuit structure includes an oxide semiconductor layer. The hydrogen sensing structure includes a hydrogen ion sensing layer containing a resistance change material, and a first sensing electrode layer and a second sensing electrode layer disposed at opposite ends of the hydrogen ion sensing layer. A bottom surface and a top surface of the hydrogen ion sensing layer are arranged to have same levels as a bottom surface and a top surface of the oxide semiconductor layer based on the surface of the substrate, respectively.

A semiconductor device according to an embodiment of the present disclosure includes an integrated circuit structure disposed over a substrate and a hydrogen sensing structure disposed over the substrate to be laterally spaced apart from the integrated circuit structure. The integrated circuit structure includes an oxide semiconductor layer. The hydrogen sensing structure includes a hydrogen ion sensing layer , and a first sensing electrode layer and a second sensing electrode layer disposed at opposite ends of the hydrogen ion sensing layer. Each of the first sensing electrode layer and the second sensing electrode layer has a work function greater than a work function of the hydrogen ion sensing layer. The hydrogen sensing structure is disposed to have a same level as the integrated circuit structure based on the surface of the substrate.

### BRIEF DESCRIPTION OF THE DRAWINGS

FIG. 1A and FIG. 1B illustrate a semiconductor device according to an embodiment of the present disclosure.
FIG. 2A to FIG. 2C illustrate an operation method of a hydrogen sensing structure according to an embodiment of the present disclosure.
FIG. 3A and FIG. 3B illustrate a cross-sectional view of a semiconductor device according to an embodiment of the present disclosure.
FIG. 4A and FIG. 4B illustrate a cross-sectional view of a semiconductor device according to an embodiment of the present disclosure.
FIG. 5A and FIG. 5B illustrate an operation method of a hydrogen sensing structure according to an embodiment of the present disclosure.
FIG. 6A and FIG. 6B illustrate a cross-sectional view of a semiconductor device according to an embodiment of the present disclosure.

### DETAILED DESCRIPTION

Hereinafter, embodiments of the present disclosure will be described in detail with reference to the accompanying drawings. In the drawings, in order to clearly express the components of each device, the sizes of the components, such as width and thickness of the components, may be enlarged. The cross-hatching throughout the figures illustrates corresponding or similar areas between the figures rather than indicating the materials associated with the areas.

The terms used herein may correspond to words selected in consideration of their functions in the embodiments, and the meanings of the terms may be construed to be different according to the ordinary skill in the art to which the embodiments belong. If expressly defined in detail, the terms may be construed according to the definitions. Unless otherwise defined, the terms (including technical and scientific terms) used herein have the same meaning as commonly understood by one of ordinary skill in the art to which the embodiments belong.

Expression of a singular form of a word should be understood to include the plural forms of the word unless clearly used otherwise in the context. It will be understood that the terms "comprise," "include," or "have" are intended to specify the presence of a feature, a number, a step, an operation, a component, an element, a part, or combinations thereof, but not used to preclude the presence or possibility of addition one or more other features, numbers, steps, operations, components, elements, parts, or combinations thereof.

Terms used in the specification of the present application are terms selected in consideration of functions in the presented embodiments, and the meaning of the terms may vary depending on the intention or custom of a user or operator in the technical field. The meanings of the terms used follow the definitions defined when specifically defined herein, and may be interpreted as meanings generally recognized by those skilled in the art in the absence of specific definitions.

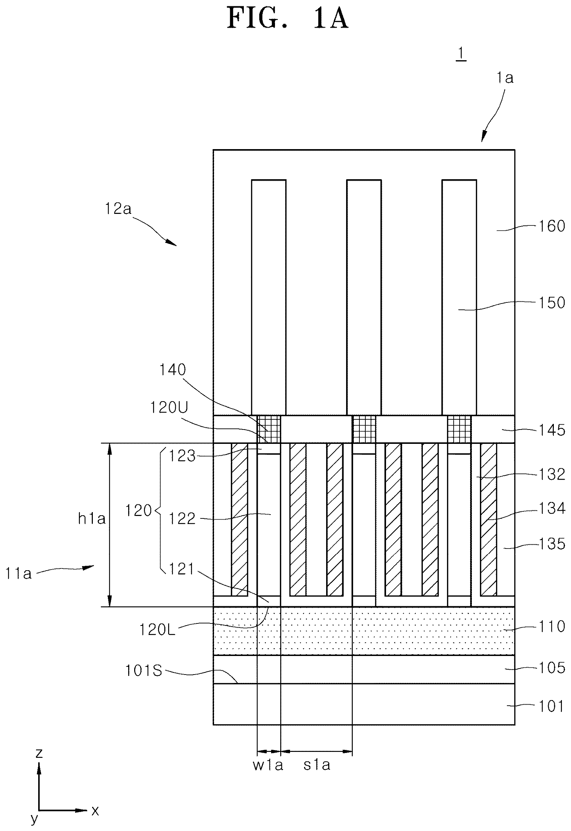

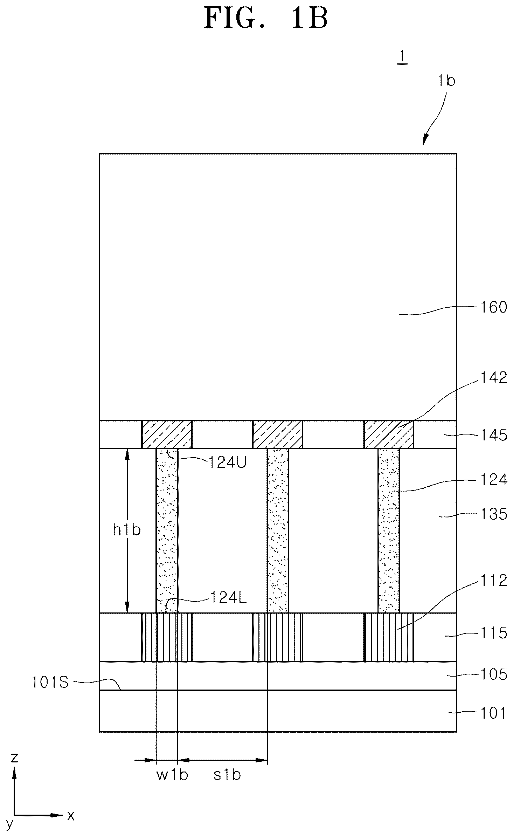

FIG. 1A and FIG. 1B illustrate cross-sectional views of portions of a semiconductor device 1 according to an embodiment of the present disclosure. Specifically, FIG. 1A illustrates a cross-sectional view of an integrated circuit structures 1a of the semiconductor device 1, and FIG. 1B illustrates a cross-sectional view of hydrogen sensing structures 1b of the semiconductor device 1. In an embodiment, the semiconductor device 1 may be a Dynamic Random Access Memory (DRAM) device.

Although the semiconductor device 1 is not shown in totality, it should be understood that the portions of the semiconductor device 1 illustrated in FIG. 1A and FIG. 1B are included in the same device, semiconductor device 1, and additional portions of the device may be arranged/disposed as understood by a person of skill in the art based on the present disclosure.

Referring to FIG. 1A and FIG. 1B, the semiconductor device 1 includes at least one integrated circuit structure 1a and at least one hydrogen sensing structure 1b that are disposed over a substrate 101. The at least one integrated circuit structure 1a and the at least one hydrogen sensing structure 1b are separated from each other in the semiconductor device 1 in a lateral direction parallel to a surface 101S of the substrate 101 (e.g., x-direction). In FIG. 1A and FIG. 1B, the semiconductor device 1 is illustrated as including three integrated circuit structures 1a and three hydrogen sensing structures 1b, but the present disclosure is not limited thereto. The semiconductor device 1 includes a plurality of cell regions and a scribe lane region between the plurality of cell regions in the substrate 101. In an embodiment, the integrated circuit structure 1a is disposed in the plurality of cell regions, and the hydrogen sensing structure 1b is disposed in a test pattern region located within the scribe lane region. In another embodiment, the integrated circuit structure 1a and the hydrogen sensing structure 1b are disposed in the test pattern region. As an example, the integrated circuit structure 1a and the hydrogen sensing structure 1b are disposed adjacent to each other in the lateral direction, for example, in the x-direction over the substrate 101.

Referring to FIG. 1A, the integrated circuit structure 1a of the semiconductor device 1 includes an oxide semiconductor layer 120. The integrated circuit structure 1a may include a plurality of conductive layers sequentially stacked from a surface 101S of the substrate 101. The oxide semiconductor layer 120 may be disposed on a lowermost conductive layer among the plurality of conductive layers.

When hydrogen ions exceeding a threshold amount are introduced into the oxide semiconductor layer 120, the hydrogen ions reduce the oxide within the oxide semiconductor layer 120, thereby changing electrical properties of the oxide semiconductor layer 120 from semiconductor properties to metallic properties. When the change in electrical properties occurs, operational reliability of the integrated circuit structure 1a that applies the oxide semiconductor layer 120 as an electrically active layer may be reduced. The semiconductor device 1 according to an embodiment of the present disclosure can determine concentration of the hydrogen ions introduced into the oxide semiconductor layer 120 of the integrated circuit structure 1a using the hydrogen sensing structure 1b. As a result, influence of the hydrogen ions on the integrated circuit structure 1a can be effectively determined.

Referring to FIG. 1A, the integrated circuit structure 1a may include a cell transistor 11a including the oxide semiconductor layer 120. The integrated circuit structure 1a may include the cell transistor 11a, a cell capacitor 12a, and a contact plug 140 that electrically connects the cell capacitor 12a to the cell transistor 11a. A plurality of cell transistors 11a may be arranged in one direction, for example, in the x-direction to form a cell array. A plurality of cell capacitors 12a may be arranged in the one direction or another direction over the cell transistors 11a.

The substrate 101 may include a conductor, a semiconductor, or an insulator on which semiconductor integration processes can be performed. In an embodiment, the substrate 101 may be a semiconductor substrate. The semiconductor substrate may be doped with an N-type dopant or a P-type dopant.

A base insulating layer 105 may be disposed on the substrate 101. The base insulating layer 105 electrically insulates the substrate 101 and a bit line 110 from each other. The base insulating layer 105 may include an insulating material. The insulating material may include, for example, oxide, nitride, oxynitride, or a combination of two or more thereof.

The bit line 110 may be a conductive line that extends along the x-direction on the base insulating layer 105. The bit line 110 may include a conductive material. The conductive material may include, for example, metal, metal nitride, metal silicide, or a combination of two or more thereof. In an embodiment, the bit line 110 may include a tungsten (W) layer, a tungsten nitride layer, or a combination of two or more thereof.

The cell transistor 11a may be disposed on the bit line 110. The cell transistor 11a may include the oxide semiconductor layer 120 that extends in a vertical direction perpendicular to the surface 101S of the substrate 101, that is, in the z-direction on the bit line 110. The oxide semiconductor layer 120 may include metal oxide. The oxide semiconductor layer 120 may have electrical semiconductor properties.

A plurality of oxide semiconductor layers 120 may be arranged in the lateral direction, for example, in the x-direction. In an embodiment, the oxide semiconductor layer 120 may have a shape of a pillar. The pillar may be a cylinder, an elliptical column, or a polygonal column. The oxide semiconductor layer 120 may have a width (or a diameter) w1a and a height h1a. The oxide semiconductor layer 120 may have a spacing s1a between adjacent oxide semiconductor layers in the lateral direction, for example, in the x-direction.

In an embodiment, the oxide semiconductor layer 120 may include a first interface layer 121, a channel layer 122, and a second interface layer 123. The channel layer 122 may be applied as a vertical channel of the cell transistor 11a. As an example, the channel layer 122 may include indium gallium zinc oxide (IGZO), indium gallium oxide (IGO), indium zinc oxide (IZO), or a combination of two or more thereof. Each of the first interface layer 121 and the second and interface layer 123 may have a lower electrical resistance than the channel layer 122. In the metal oxide contained in each of the first interface layer 121 and the second and interface layer 123, a metal content may be greater than an oxygen content in terms of the stoichiometric ratio. Each of the first interface layer 121 and the second interface layer 123 may include, for example, indium-rich indium gallium zinc oxide (IGZO). In the indium-rich indium gallium zinc oxide (IGZO), the indium content among metal components constituting the indium gallium zinc oxide may be relatively large, and the contents of gallium (Ga) and zinc (Zn) may be relatively small. The first interface layer 121 may reduce a contact resistance between the bit line 110 and the channel layer 122. The second interface layer 123 may reduce the contact resistance between the channel layer 122 and the contact plug 140.

Referring to FIG. 1A, a gate dielectric layer 132 in contact with a side wall surface of the oxide semiconductor layer 120 is disposed on the bit line 110. In an embodiment, the gate dielectric layer 132 may be disposed to surround the oxide semiconductor layer 120. The gate dielectric layer 132 may include, for example, oxide, nitride, oxynitride, or a combination of two or more thereof. A word line 134 that functions as a gate electrode layer may be disposed on the gate dielectric layer 132. The word line 134 may be a conductive line that extends in one direction, for example, in the y-direction. The word line 134 may be disposed to surround the oxide semiconductor layer 120 with the gate dielectric layer 132 therebetween. The word line 134 may include a conductive material. The conductive material may include, for example, metal, metal nitride, metal silicide, or a combination of two or more thereof. In an embodiment, the word line 134 may include a tungsten layer, a tungsten nitride layer, or a combination of two or more thereof. A second interlayer insulating layer 135 may be disposed in a space between the word lines 134 arranged in the lateral direction, for example, in the x-direction. The second interlayer insulating layer 135 may include, for example, oxide, nitride, oxynitride, or a combination of two or more thereof.

Referring to FIG. 1A, a third interlayer insulating layer 145 may be disposed over the cell transistor 11a. The third interlayer insulating layer 145 may include, for example, oxide, nitride, oxynitride, or a combination of two or more thereof. The contact plug 140 may be disposed in the third interlayer insulating layer 145. The contact plug 140 may be electrically connected to the second interface layer 123. The contact plug 140 may include a conductive material. The conductive material may include, for example, metal, metal nitride, metal silicide, or a combination of two or more thereof.

Referring to FIG. 1A, the cell capacitor 12a is disposed over the cell transistor 11a. In an embodiment, the cell capacitor 12a may include a capacitor structure 150 disposed on the contact plug 140. The capacitor structure 150 may include a storage node electrode layer, a capacitor dielectric layer, and a plate electrode layer. The storage node electrode layer may be electrically connected to the contact plug 140. When the cell transistor 11a is turned on, electric charges supplied from the bit line 110 are stored in the capacitor dielectric layer via the channel layer 122, the contact plug 140, and the storage node electrode layer. A fourth interlayer insulating layer 160 that buries the capacitor structure 150 on the third interlayer insulating layer 145 may be disposed.

Referring to FIG. 1B, the hydrogen sensing structure 1b is provided. The hydrogen sensing structure 1b may be disposed to be separated and spaced apart from the integrated circuit structure 1a in FIG. 1A in the semiconductor device 1 in the lateral direction, for example, in the x-direction or the y-direction. The hydrogen sensing structure 1b may include a first sensing electrode layer 112, a hydrogen ion sensing layer 124, and a second sensing electrode layer 142 that are sequentially disposed in the z-direction over the substrate 101. The first sensing electrode layer 112 and the second sensing electrode layer 142 may be respectively disposed at opposite ends of the hydrogen ion sensing layer 124 in the vertical direction.

Referring to FIG. 1A and FIG. 1B, the hydrogen sensing structure 1b is located at substantially the same level as the integrated circuit structure 1a from the substrate 101. The first sensing electrode layer 112 may be disposed on the base insulating layer 105. The first sensing electrode layer 112 may be disposed on substantially the same plane as the bit line 110 of the integrated circuit structure 1a in FIG. 1A. The first interlayer insulating layer 115 may be disposed between the first sensing electrode layers 112 that are adjacent to each other in the lateral direction. The first interlayer insulating layer 115 may include, for example, oxide, nitride, oxynitride, or a combination of two or more thereof.

The hydrogen ion sensing layer 124 is disposed on the first sensing electrode layer 112. The hydrogen ion sensing layer 124 extends in the vertical direction. In an embodiment, the hydrogen ion sensing layer 124 may be disposed on substantially the same plane as the oxide semiconductor layer 120 of the integrated circuit structure 1a in FIG. 1A. In an embodiment, the hydrogen ion sensing layer 124 may have substantially the same shape as the oxide semiconductor layer 120 in FIG. 1A. As an example, the hydrogen ion sensing layer 124 may have a shape of a pillar extending in the vertical direction perpendicular to the surface 101S of the substrate 101. The pillar may be a cylinder, an elliptical pillar, or a polygonal pillar. A plurality of hydrogen ion sensing layers 124 may be disposed in one direction, for example, in the x-direction, to form an array.

The hydrogen ion sensing layer 124 may have a width (or diameter) w1b and a height h1b. The hydrogen ion sensing layer 124 may have a spacing s1b between other adjacent hydrogen ion sensing layers in the lateral direction, for example, in the x-direction. The diameter w1b and the height h1b of the hydrogen ion sensing layer 124 may be substantially the same as the diameter w1a and the height h1a of the oxide semiconductor layer 120 in FIG. 1A, respectively. The spacing s1b between the hydrogen ion sensing layers 124 adjacent to each other in the lateral direction, for example, in the x-direction, may be substantially the same as the spacing s1b between the oxide semiconductor layer 120 adjacent to each other in the lateral direction, for example, in the x-direction, shown in FIG. 1A. The second interlayer insulating layer 135 may be disposed between the adjacent hydrogen ion sensing layers 124. The second interlayer insulating layer 135 may include, for example, oxide, nitride, oxynitride, or a combination of two or more thereof. As described above, in an embodiment, a bottom surface 124L and a top surface 124U of the hydrogen ion sensing layer 124 shown in FIG. 1B is located at substantially the same level as a bottom surface 120L and a top surface 120U of the oxide semiconductor layer 120 shown in FIG. 1A, respectively.

The second sensing electrode layer 142 is disposed on the hydrogen ion sensing layer 124. The second sensing electrode layer 142 may be disposed on substantially the same plane as the contact plug 140 in FIG. 1A. The third interlayer insulating layer 145 may be disposed between the second sensing electrode layers 142 that are adjacent in the lateral direction. A fourth interlayer insulating layer 160 may be disposed on the second sensing electrode layer 142 and the third interlayer insulating layer 145. Each of the third interlayer insulating layer 145 and the fourth interlayer insulating layer 160 may include, for example, oxide, nitride, oxynitride, or a combination of two or more thereof. The second interlayer insulating layer 135 to the fourth interlayer insulating layer 160 may isolate the hydrogen ion sensing layer 124 from an external air environment. That is, each of the second interlayer insulating layer 135 to the fourth interlayer insulating layer 160 functions as an embedding layer for the hydrogen ion sensing layer 124.

Referring to FIG. 1B, the hydrogen ion sensing layer 124 includes a resistance change material. One of the first sensing electrode layer 112 and the second sensing electrode layer 142 may be an active electrode layer, and the other may be an inactive electrode layer. In an embodiment, an electrical resistance state of the hydrogen ion sensing layer 124 may be switched from a high resistance state to a low resistance state by a forming voltage or a set voltage applied between the first sensing electrode layer 112 and the second sensing electrode layer 142. Even after the forming voltage or the set voltage is removed, the hydrogen ion sensing layer 124 may maintain the switched low resistance state. In another embodiment, the electrical resistance state of the hydrogen ion sensing layer 124 may be switched from the low resistance state to the high resistance state by a reset voltage applied between the first sensing electrode layer 112 and the second sensing electrode layer 142. Even after the reset voltage is removed, the hydrogen ion sensing layer 124 can maintain the switched high resistance state.

In an embodiment, the hydrogen ion sensing layer 124 has conductivity to metal ions. That is, the hydrogen ion sensing layer 124 may allow conduction of metal ions through the inside of the hydrogen ion sensing layer 124. The hydrogen ion sensing layer 124 may include, for example, tantalum oxide, aluminum oxide, lithium nitride, or a combination of two or more thereof. The active electrode layer may include, for example, copper (Cu), lithium (Li), or a combination thereof. The inactive electrode layer may include, for example, platinum (Pt), gold (Au), iridium (Ir), titanium nitride, or a combination of two or more thereof.

Each of the first sensing electrode layer 112 and the second sensing electrode layer 142 may be electrically connected to a driving circuit that senses hydrogen ions. When the forming voltage or the set voltage is applied between the first sensing electrode layer 112 and the second sensing electrode layer 142 through the driving circuit, the active electrode layer that is one of the first sensing electrode layer 112 and the second sensing electrode layer 142 provides metal ions to the hydrogen ion sensing layer 124. Within the hydrogen ion sensing layer 124, the metal ions may be aggregated along an electric field formed by the forming voltage or the set voltage, and thus a conductive filament of metal may be formed. The conductive filament electrically may connect the first sensing electrode layer 112 and the second sensing electrode layer 142, so that the electrical resistance state of the hydrogen ion sensing layer 124 may be switched from the high resistance state to the low resistance state. Conversely, when the reset voltage is applied between the first sensing electrode layer 112 and the second sensing electrode layer 142 through the driving circuit, the metal may be separated from the conductive filament, thereby disconnecting the conductive filament. By disconnection of the conductive filament, electrical connection between the first sensing electrode layer 112 and the second sensing electrode layer 142 may be disconnected. As a result, the electrical resistance state of the hydrogen ion sensing layer 124 may be switched from the low resistance state to the high resistance state.

In another embodiment, the hydrogen ion sensing layer 124 may include oxygen vacancies. The hydrogen ion sensing layer 124 may include, for example, hafnium oxide, tantalum oxide, titanium oxide, nickel oxide, zirconium oxide, aluminum oxide, or a combination of two or more thereof. The active electrode layer may include, for example, titanium (Ti), tantalum (Ta), aluminum (Al), hafnium (Hf), or a combination of two or more thereof. The inactive electrode layer may include, for example, platinum (Pt), gold (Au), iridium (Ir), titanium nitride, or a combination of two or more thereof.

When the forming voltage or the set voltage is applied between the first sensing electrode layer 112 and the second sensing electrode layer 142 through the driving circuit, the active electrode layer may provide the oxygen vacancies to the hydrogen ion sensing layer 124. Within the hydrogen ion sensing layer 124, the oxygen vacancies are aggregated along an electric field formed by the forming voltage or the set voltage, so that a conductive filament of the oxygen vacancies may be formed. As the conductive filament electrically connects the first sensing electrode layer 112 and the second sensing electrode layer 142, the electrical resistance state of the hydrogen ion sensing layer 124 may be switched from the high resistance state to the low resistance state. Conversely, when the reset voltage is applied between the first sensing electrode layer 112 and the second sensing electrode layer 142 through the driving circuit, the oxygen vacancies are separated from the conductive filament, thereby disconnecting the conductive filament. By disconnection of the conductive filament, the electrical resistance state of the hydrogen ion sensing layer 124 may be switched from the low resistance state to the high resistance state. As described above, when an external stimulus such as voltage is applied to the hydrogen ion sensing layer 124, the electrical resistance state of the hydrogen ion sensing layer 124 may be changed.

When the hydrogen ion sensing layer 124 is exposed to hydrogen ions, the above-described electrical resistance characteristics of the hydrogen ion sensing layer 124 may change. As will be described later, the concentrations of hydrogen ions distributed near the oxide semiconductor layer may be detected by observing the change in the electrical properties of the hydrogen ion sensing layer 124.

In some embodiments, the hydrogen sensing structure 1b may include an array of hydrogen ion sensing layers 124 arranged in one direction, for example, the x-direction. As an example, the hydrogen sensing structure 1b may include only an array of the hydrogen ion sensing layers 124 configured to form the conductive filament of metal. As another example, the hydrogen sensing structure 1b may include only an array of the hydrogen ion sensing layers 124 configured to form the conductive filament of oxygen vacancies. As another example, the hydrogen sensing structure 1b may include a first array of the hydrogen ion sensing layers 124 configured to form the conductive filament of metal and a second array of the hydrogen ion sensing layers 124 configured to form the conductive filament of oxygen vacancies.

Hereinafter, a method of sensing hydrogen ions is described in detail using a characteristic in which the electrical resistance of the hydrogen ion sensing layer changes according to an inflow amount of hydrogen ions.

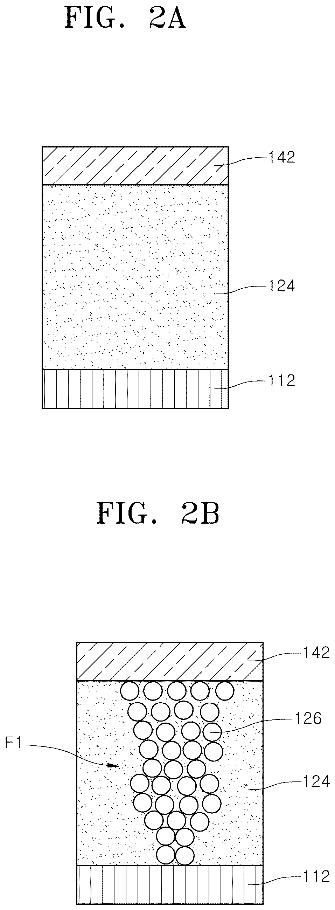

FIG. 2A to FIG. 2C illustrate an operation method of a hydrogen sensing structure according to an embodiment of the present disclosure. The hydrogen sensing structure may be the hydrogen sensing structure 1b of the semiconductor device 1 described with reference to FIG. 1B.

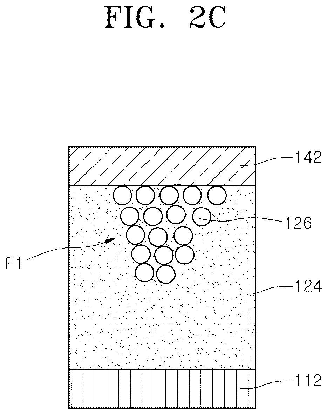

FIG. 2A illustrates a first state of a hydrogen sensing layer 124 before an initial forming operation for the hydrogen sensing structure 1b is performed. FIG. 2B illustrates a second state of the hydrogen ion sensing layer 124 immediately after a set operation for the hydrogen sensing structure 1b is completed. FIG. 2C illustrates a third state of the hydrogen ion sensing layer 124 immediately after a reset operation for the hydrogen sensing structure 1b is completed.

Referring to FIG. 2A, the first state of the hydrogen ion sensing layer 124 indicates a high resistance state. When the hydrogen ion sensing layer 124 is exposed to hydrogen ions in the first state, the electrical characteristics of the hydrogen ion sensing layer 124 may deteriorate. For example, when a voltage is applied between a first sensing electrode 112 and a second sensing electrode 142 in the first state, a leakage current output from the hydrogen sensing structure 1b may be increased in proportion to concentration of the hydrogen ions, compared to a case where the hydrogen ion sensing layer 124 is not exposed to the hydrogen ions. As a result, when the hydrogen ion sensing layer 124 is in the first state, the concentration of the hydrogen ions flowing into the hydrogen ion sensing layer 124 can be determined based on a magnitude of the output leakage current.

Referring to FIG. 2B, the second state of the hydrogen ion sensing layer 124 indicates a low resistance state that is formed by the set operation. In the second state, a conductive filament F1 may be formed by a metal 126 (or an oxygen vacancy) to connect the first sensing electrode layer 112 and the second sensing electrode layer 142. When the hydrogen ion sensing layer 124 is exposed to the hydrogen ions in the second state, the resistance switching characteristics of the hydrogen ion sensing layer 124 may change during a subsequent reset operations. That is, the reset operation may be performed on the hydrogen ion sensing layer 124 in the second state by applying a voltage while sweeping between the first sensing electrode layer 112 and the second sensing electrode layer 142. Sweeping between two electrode layers in this context refers to a potential sweep, in which the potential of the working electrode is varied linearly in time between two specific voltage values, such as applied for electrochemical measurement processes like linear sweep voltammetry (LSV), cyclic voltammetry (CV), etc. When the hydrogen ion sensing layer 124 is exposed to the hydrogen ions, a rapid increase in the resistance, that is, a rapid decrease in output current may occur at the reset voltage, compared to when the hydrogen ion sensing layer 124 is not exposed to the hydrogen ions. This may mean that the resistance switching does not occur gradually over a voltage range, but rather occurs abruptly at the reset voltage.

During the reset operation, electrochemical reactions may occur between the hydrogen ions and the metal 126 (or the oxygen vacancy) of the conductive filament F1, so that detachment of the metal 126 (or the oxygen vacancy) from the conductive filament F1 may be accelerated. As a result, resistance switching reactions from the low resistance state to the high resistance state may occur rapidly at the reset voltage. When the hydrogen ion sensing layer 124 is exposed to the hydrogen ions exceeding a threshold concentration, disconnections of the conductive filament F1 may occur without application of an external voltage, thereby causing the resistance switching to the high resistance state regardless of the reset operation. As a result, when the hydrogen ion sensing layer 124 is in the second state, the concentration of the hydrogen ions flowing into the hydrogen ion sensing layer 124 can be determined based on a type of the resistance switching and a degree of change in magnitude of the output current during the reset operation.

Referring to FIG. 2C, the third state of the hydrogen ion sensing layer 124 indicates a high resistance state that is formed by the reset operation. In the third state, the disconnected conductive filament F1 may exist between the first sensing electrode layer 112 and second sensing electrode layer 142 due to the reset operation. In this instance, the disconnected conductive filament F1 may be in contact with the second sensing electrode layer 142 and not in contact with the first sensing electrode layer 112. When the hydrogen ion sensing layer 124 is exposed to the hydrogen ions in the third state, the resistance switching characteristics of the hydrogen ion sensing layer 124 may change during a subsequent set operation.. As an example, the set operation may be performed on the hydrogen ion sensing layer 124 in the third state by applying a voltage while sweeping between the first sensing electrode layer 112 and the second sensing electrode layer 142. When the hydrogen ion sensing layer 124 is exposed to the hydrogen ions, a rapid decrease in the resistance, that is, a rapid increase in the output current may occur at the set voltage, compared to when the hydrogen ion sensing layer 124 is not exposed to the hydrogen ions. This may mean that the resistance switching does not occur gradually over a voltage range, but rather occurs abruptly at the set voltage.

During the set operation, electrochemical reactions may occur between the hydrogen ions flowing into the hydrogen ion sensing layer 124 and the metal 126 (or the oxygen vacancy) of the disconnected conductive filament F1. By the electrochemical reactions, at the set voltage, movement of the metal 126 (or the oxygen vacancy) inside the hydrogen ion sensing layer 124 to the disconnected conductive filament F1 may be accelerated. As a result, a resistance switching reaction may occur rapidly from the high resistance state to the low resistance state. As a result, when the hydrogen ion sensing layer 124 is in the third state, the concentration of the hydrogen ions flowing into the hydrogen ion sensing layer 124 can be determined based on the type of the resistance switching and the degree of change in the magnitude of the output current during the set operation.

According to an embodiment of the present disclosure, the concentration of the hydrogen ions flowing into the hydrogen ion sensing layer can be determined based on a change in the electrical resistance of the hydrogen ion sensing layer. The concentration of the hydrogen ions flowing into an oxide semiconductor layer of an integrated circuit structure can be determined through the concentration of the hydrogen ions flowing into the hydrogen ion sensing layer. As a result, influence of the hydrogen ions on the electrical properties of the integrated circuit structure can be effectively determined.

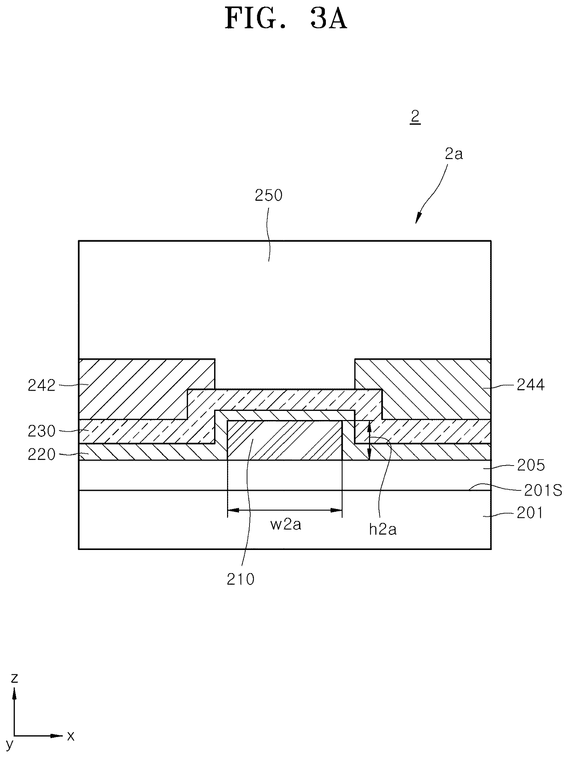

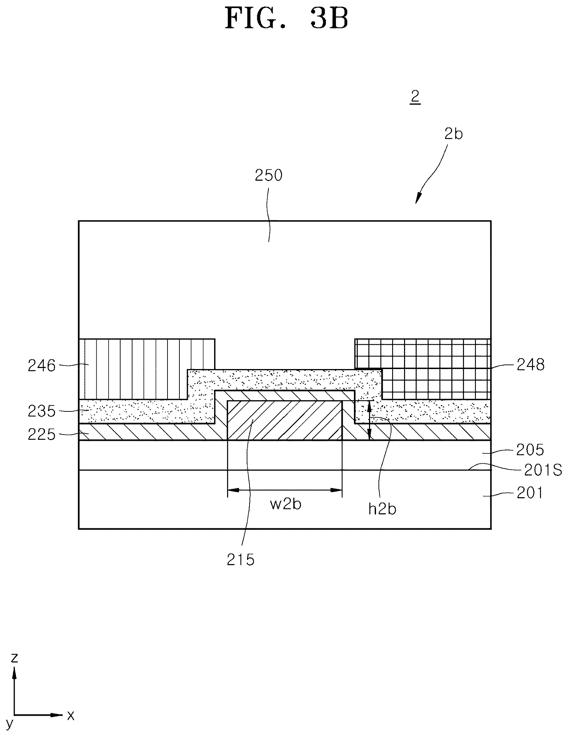

FIG. 3A and FIG. 3B illustrate cross-sectional views of a semiconductor device 2 according to an embodiment of the present disclosure. Specifically, FIG. 3A illustrates a cross-sectional view of an integrated circuit structure 2a of the semiconductor device 2, and FIG. 3B illustrates a cross-sectional view of a hydrogen sensing structure 2b of the semiconductor device 2.

Referring to FIG. 3A and FIG. 3B, the semiconductor device 2 includes the integrated circuit structure 2a and the hydrogen sensing structure 2b that are disposed over a substrate 201. The integrated circuit structure 2a and the hydrogen sensing structure 2b are separated from each other in a lateral direction parallel to a surface 201S of the substrate 201. The semiconductor device 2 includes a plurality of cell regions and a scribe lane region between the plurality of cell regions in the substrate 201. In an embodiment, the integrated circuit structure 2a may be disposed in the plurality of cell regions, and the hydrogen sensing structure 2b is disposed in a test pattern region located in the scribe lane region of semiconductor device 2. In another embodiment, both the integrated circuit structure 2a and the hydrogen sensing structure 2b may be disposed in the test pattern region of semiconductor device 2.

Referring to FIG. 3A, the integrated circuit structure 2a may include a field effect transistor including an oxide semiconductor layer 230. Depending on the concentration of hydrogen ions flowing into the oxide semiconductor layer 230, electrical properties of the oxide semiconductor layer 230 may be changed from semiconductor properties to metal properties. The semiconductor device 2 according to an embodiment of the present disclosure can determine the concentration of hydrogen ions flowing into the oxide semiconductor layer 230 of the integrated circuit structure 2a using the hydrogen sensing structure 2b.

Referring to FIG. 3A, the substrate 201 may include a conductor, a semiconductor, or an insulator on which a semiconductor integration processes can be performed. The substrate 201 may be substantially the same as the substrate 101 described with reference to FIG. 1A and FIG. 1B.

A base insulating layer 205 may be disposed on the substrate 201. The base insulating layer 205 may include, for example, oxide, nitride, oxynitride, or a combination of two or more thereof.

A gate electrode layer 210 may be disposed on the base insulating layer 205. The gate electrode layer 210 may include a conductive material. The conductive material may include, for example, doped semiconductor, metal, metal nitride, metal silicide, or a combination of two or more thereof. The gate electrode layer 210 has a width w2a along the x-direction and a height h2a along the z-direction.

A gate dielectric layer 220 may be disposed on the gate electrode layer 210. The gate dielectric layer 220 may be disposed on the base insulating layer 205 to cover the gate electrode layer 210. The gate dielectric layer 220 may include, for example, oxide, nitride, oxynitride, or a combination of two or more thereof.

The oxide semiconductor layer 230 may be disposed on the gate dielectric layer 220. The oxide semiconductor layer 230 may function as a channel layer of the field effect transistor. The oxide semiconductor layer 230 may have a uniform thickness. The material and electrical properties of the oxide semiconductor layer 230 may be substantially the same as the material and electrical properties of the oxide semiconductor layer 120 described with reference to FIG. 1A and FIG. 1B, respectively.

A source electrode layer 242 and a drain electrode layer 244 may be disposed to be spaced apart from each other on the oxide semiconductor layer 230. Each of the source electrode layer 242 and the drain electrode layer 244 may include a conductive material. The conductive material may include, for example, doped semiconductor, metal, metal nitride, metal silicide, or a combination of two or more thereof.

Referring to FIG. 3A, an embedding layer 250 covering the oxide semiconductor layer 230, the source electrode layer 242, and the drain electrode layer 244 may be disposed. The embedding layer 250 may include, for example, oxide, nitride, oxynitride, or a combination of two or more thereof.

Referring to FIG. 3B, the hydrogen sensing structure 2b may be provided. The hydrogen sensing structure 2b may be disposed to be separated and spaced apart from the integrated circuit structure 2a of FIG. 3A in a lateral direction parallel to a surface 201S of the substrate 201, for example, in the x-direction or the y-direction. The hydrogen sensing structure 2b may include a hydrogen ion sensing layer 235 disposed over the substrate 201, and a first sensing electrode layer 246 and a second sensing electrode layer 248 that are disposed on the hydrogen ion sensing layer 235. The first sensing electrode layer 246 and the second sensing electrode layer 248 may be disposed spaced apart from each other in the lateral direction, for example, in the x-direction.

A first buffer layer 215 may be disposed on the base insulating layer 205. The first buffer layer 215 may have substantially the same shape as the gate electrode layer 210 of FIG. 3A. The first buffer layer 215 has a width w2b along the x-direction and a height h2b along the z-direction. The width w2b and height h2b of the first buffer layer 215 may be substantially the same as the width w2a and height h2a of the gate electrode layer 210 of FIG. 3A, respectively. The first buffer layer 215 may include, for example, oxide, nitride, oxynitride, or a combination of two or more thereof.

A second buffer layer 225 covering the first buffer layer 215 may be disposed on the base insulating layer 205. The second buffer layer 225 may have a uniform thickness. The thickness of the second buffer layer 225 may be substantially the same as the thickness of the gate dielectric layer 220 of FIG. 3A. A material of the second buffer layer 225 may be substantially the same as the material of the gate dielectric layer 220 of FIG. 3A.

The hydrogen ion sensing layer 235 may be disposed on the second buffer layer 225. The material and electrical properties of the hydrogen ion sensing layer 235 may be substantially the same as the material and electrical properties of the hydrogen ion sensing layer 124 described with respect to FIG. 1B. A thickness and a profile of the hydrogen ion sensing layer 235 may be substantially the same as the thickness and profile of the oxide semiconductor layer 230 of FIG. 3A.

The first sensing electrode layer 246 and the second sensing electrode layer 248 may be disposed on the hydrogen ion sensing layer 235. The first sensing electrode layer 246 and the second sensing electrode layer 248 may be disposed to be spaced apart from each other in the lateral direction, for example, in the x-direction. The material and electrical properties of each of the first sensing electrode layer 246 and the second sensing electrode layer 248 may be substantially the same as the material and electrical properties of each of the first sensing electrode layer 112 and the second sensing electrode layer 142 described with reference to FIG. 1B. A thickness of each of the first sensing electrode layer 246 and the second sensing electrode layer 248 may be substantially the same as the thickness of each of the source electrode layer 242 and the drain electrode layer 244 described with reference to FIG. 3A.

The embedding layer 250 may be disposed to embed the hydrogen ion sensing layer 235 and the first sensing electrode layer 246 and the second sensing electrode layer 248. The embedding layer 250 may isolate the hydrogen ion sensing layer 235, the first sensing electrode layer 246, and the second sensing electrode layer 248 from an external air environment.

An operation method of the hydrogen sensing structure 2b is substantially the same as the operation method of the hydrogen sensing structure 1b described with reference to FIG. 2A to FIG. 2C. Based on the change in the electrical resistance properties of the hydrogen ion sensing layer 235 of the hydrogen sensing structure 2b, the concentration of the hydrogen ions flowing into the oxide semiconductor layer 230 of the integrated circuit structure 2a can be effectively determined.

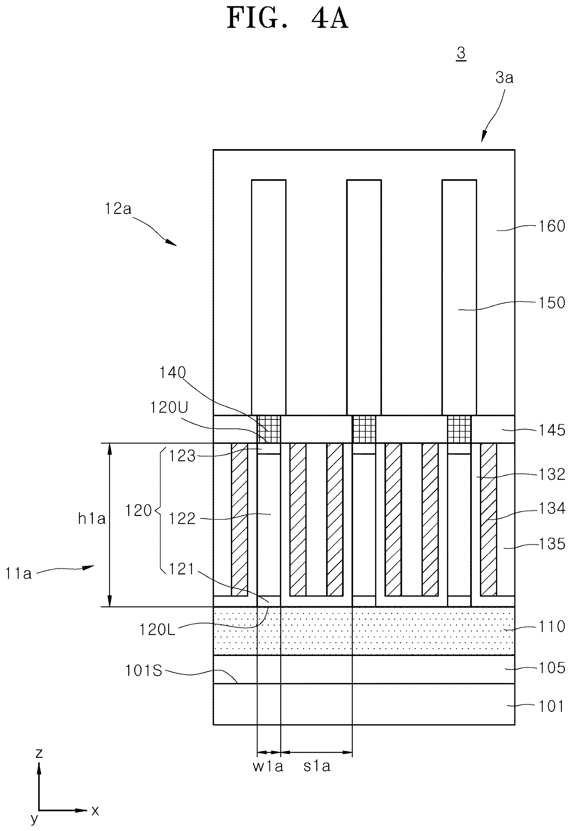

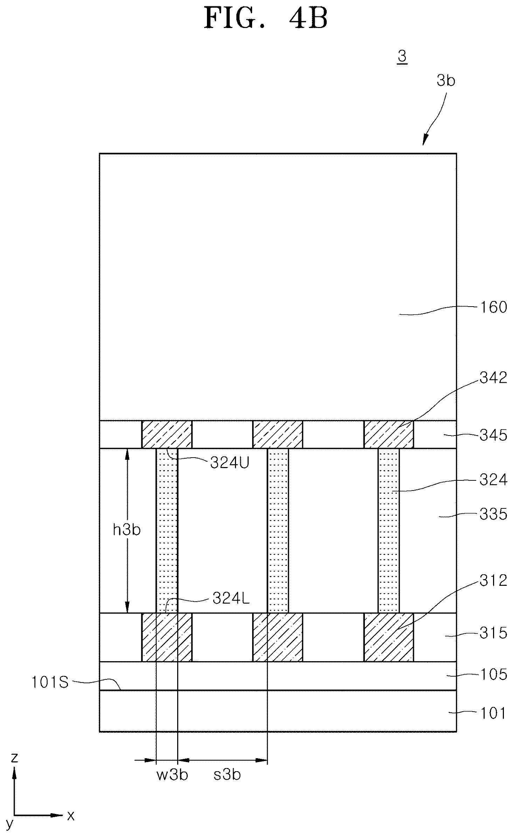

FIG. 4A and FIG. 4B illustrate cross-sectional views of a semiconductor device 3 according to an embodiment of the present disclosure. Specifically, FIG. 4A illustrates a cross-sectional view of an integrated circuit structure 3a of the semiconductor device 3, and FIG. 4B illustrates a cross-sectional view of a hydrogen sensing structure 3b of the semiconductor device 3.

Referring to FIG. 4A and FIG. 4B, the semiconductor device 3 includes, respectively shown with respect to FIG. 4A and FIG. 4B, the integrated circuit structure 3a and the hydrogen sensing structure 3b that are disposed over a substrate 101. The integrated circuit structure 3a and the hydrogen sensing structure 3b are separated from each other in a lateral direction parallel to a surface 101S of the substrate 101. In FIG. 4A and FIG. 4B, the semiconductor device 3 is illustrated as including three integrated circuit structures 3a and three hydrogen sensing structures 3b, but the present disclosure is not limited thereto. The semiconductor device 3 includes a plurality of cell regions and a scribe lane region between the plurality of cell regions. In an embodiment, the integrated circuit structure 3a may be disposed in the plurality of cell regions, and the hydrogen sensing structure 3b may be disposed in a test pattern region located within the scribe lane region of semiconductor device 3. In another embodiment, the integrated circuit structure 3a and the hydrogen sensing structure 3b may be disposed in the test pattern region of semiconductor device 3. As an example, the hydrogen structure 3a and the hydrogen sensing structure 3b may be disposed adjacent to each other in a lateral direction, for example, in the x-direction.

Referring to FIG. 4A, the integrated circuit structure 3a may be substantially the same as the integrated circuit structure 1a of the semiconductor device 1 described with reference to FIG. 1A. That is, the integrated circuit structure 3a may include a cell transistor 11a including an oxide semiconductor layer 120. The integrated circuit structure 3a may include the cell transistor 11a, a cell capacitor 12a, and a contact plug 140 electrically connecting the cell transistor 11a and the cell capacitor 12a.

Referring to FIG. 4B, the hydrogen sensing structure 3b may be disposed spaced apart and separated from the integrated circuit structure 3a of FIG. 4A in the lateral direction, for example, in the x-direction or y-direction. The hydrogen sensing structure 3b may include a first sensing electrode layer 312, a hydrogen ion sensing layer 324, and a second sensing electrode layer 342 that are sequentially disposed over the substrate 101. The first sensing electrode layer 312 and the second sensing electrode layer 342 may be respectively disposed at opposite ends of the hydrogen ion sensing layer 324 in a vertical direction perpendicular to the surface 101S of the substrate 101. As described below, the hydrogen sensing structure 3b may have a first Schottky junction formed at an interface between the first sensing electrode layer 312 and the hydrogen ion sensing layer 324, and a second Schottky junction formed at an interface between the second sensing electrode layer 342 and the hydrogen ion sensing layer 324.

Referring to FIG. 4A and FIG. 4B, the hydrogen sensing structure 3b may be disposed at substantially the same level as the integrated circuit structure 3a from the substrate 101. The first sensing electrode layer 312 may be disposed on the base insulating layer 105. That is, the first sensing electrode layer 312 may be disposed at substantially the same plane as a bit line 110 of FIG. 4A. A first interlayer insulating layer 315 may be disposed between the first sensing electrode layers 312 that are adjacent in the lateral direction, for example, in the x-direction. The first interlayer insulating layer 315 may include, for example, oxide, nitride, oxynitride, or a combination of two or more thereof.

The hydrogen ion sensing layer 324 may be disposed on the first sensing electrode layer 312. In an embodiment, the hydrogen ion sensing layer 324 may have a shape of a pillar extending in the vertical direction. The pillar may be a cylinder, an elliptical pillar, or a polygonal pillar. A plurality of hydrogen ion sensing layers 324 may be disposed in one direction, for example, in the x-direction, to form an array.

In an embodiment, the hydrogen ion sensing layer 324 may be disposed on substantially the same plane as the oxide semiconductor layer 120 of FIG. 4A. In an embodiment, the hydrogen ion sensing layer 324 may have substantially the same shape as the oxide semiconductor layer 120 of FIG. 4A. The hydrogen ion sensing layer 324 has a diameter or width w3b and a height h3b. The hydrogen ion sensing layer 324 has a spacing s3b between other adjacent hydrogen ion sensing layers in the lateral direction, for example, the x-direction. The diameter w3b and the height h3b of the hydrogen ion sensing layer 324 may be substantially the same as the diameter w1a and the height h1a of the oxide semiconductor layer 120 of FIG. 4A, respectively. The spacing s3b between the adjacent hydrogen ion sensing layers 324 in the lateral direction, for example, in the x-direction may be substantially the same as the spacing s1b between the adjacent oxide semiconductor layers 120 in the lateral direction, for example, in the x-direction as illustrated in FIG. 4A. A second interlayer insulating layer 335 may be disposed between the adjacent hydrogen ion sensing layers 324 in the lateral direction, for example, in the x-direction. The second interlayer insulating layer 335 may include, for example, oxide, nitride, oxynitride, or a combination of two or more thereof. As described above, in an embodiment, a bottom surface 324L and a top surface 324U of the hydrogen ion sensing layer 324 shown in FIG. 4B is located at substantially the same level as a bottom surface 120L and a top surface 120U of the oxide semiconductor layer 120 shown in FIG. 4A, respectively.

The second sensing electrode layer 342 may be disposed on the hydrogen ion sensing layer 324. The second sensing electrode layer 342 may be disposed on substantially the same plane as the contact plug 140 of FIG. 4A. A third interlayer insulating layer 345 may be disposed between the second sensing electrode layers 342 that are adjacent in the lateral direction, for example, in the x-direction. A fourth interlayer insulating layer 160 may be disposed on the second sensing electrode layer 342 and the third interlayer insulating layer 345. Each of the third interlayer insulating layer 345 and the fourth interlayer insulating layer 160 may include, for example, oxide, nitride, oxynitride, or a combination of two or more thereof. The second interlayer insulating layer 335, the third interlayer insulating layer 345, and the fourth interlayer insulating layer 160 may isolate the hydrogen ion sensing layer 324 from an external air environment. That is, each of the second interlayer insulating layer 335, the third interlayer insulating layer 345, and the fourth interlayer insulating layer 160 may function as an embedding layer.

In an embodiment, each of the first sensing electrode layer 312 and the second sensing electrode layer 342 may have a work function greater than the work function of the hydrogen ion sensing layer 324. Accordingly, each of the first sensing electrode layer 312 and the second sensing electrode layer 342 may be configured to form a Schottky junction with the hydrogen ion sensing layer 324. Each of the first sensing electrode layer 312 and the second sensing electrode layer 342 may have a work function of 5 eV or greater. In an embodiment, the first sensing electrode layer 312 and the second sensing electrode layer 342 may have different work functions. Accordingly, an energy barrier height of the first Schottky junction formed between the first sensing electrode layer 312 and the hydrogen ion sensing layer 324 may be different from the energy barrier height of the second Schottky junction formed between the second sensing electrode layer 342 and the hydrogen ion sensing layer 324.

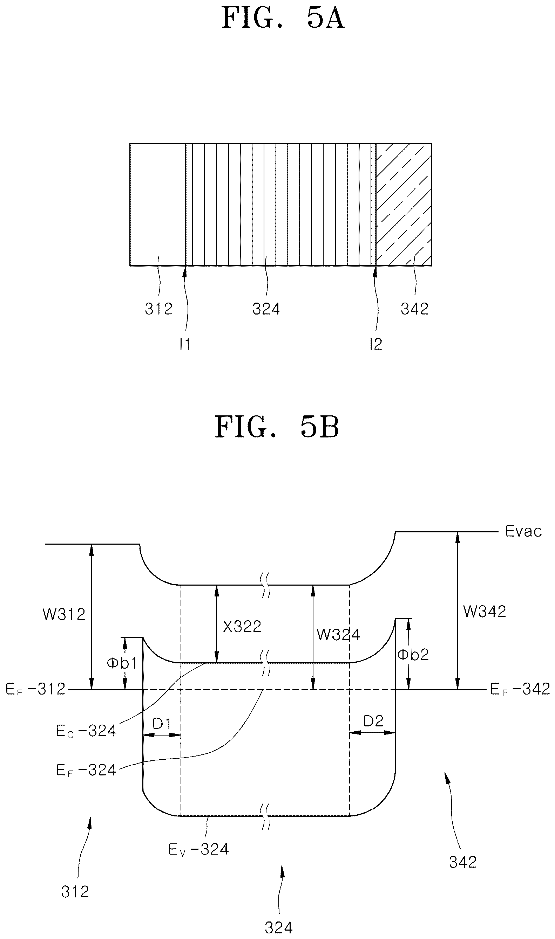

According to an embodiment of the present disclosure, when hydrogen ions are introduced into the hydrogen sensing structure 3b, at least one of the energy barrier heights of the first Schottky junction and the second Schottky junction may be changed, as described below with reference to FIG. 5A and FIG. 5B. Based on the change in the energy barrier height of the Schottky junction, the concentration of the hydrogen ions flowing into the oxide semiconductor layer 120 of the integrated circuit structure 4a can be determined.

In an embodiment, each of the first sensing electrode layer 312 and the second sensing electrode layer 342 may include, for example, platinum (Pt), palladium (Pd), silver oxide, or a combination of two or more thereof. The hydrogen ion sensing layer 324 may have a work function smaller than the work functions of the first sensing electrode layer 312 and the second sensing electrode layer 342. The hydrogen ion sensing layer 324 may include an oxide semiconductor. The oxide semiconductor may have n-type semiconductor properties. The hydrogen ion sensing layer 324 may include, for example, gallium-rich indium gallium zinc oxide (Ga-rich IGZO), gallium oxide (Ga₂O₃), mixed anion zinc oxide (MAZO), or a combination of two or more thereof.

Hereinafter, a method of sensing hydrogen ions using a characteristic in which the energy barrier height of the Schottky junction changes according to an inflow amount of the hydrogen ions is described in more detail.

FIG. 5A and FIG. 5B illustrate an operation method of a hydrogen sensing structure according to an embodiment of the present disclosure. FIG. 5A is a schematic diagram of the hydrogen sensing structure, and FIG. 5B is an energy band diagram of the hydrogen sensing structure. The operation method of the hydrogen sensing structure of FIG. 5A and FIG. 5B may be described using the hydrogen sensing structure 3b of the semiconductor device 3 described with reference to FIG. 4B.

Referring to FIG. 5A, the first sensing electrode layer 312 and the hydrogen ion sensing layer 324 may form a first interface I1. The second sensing electrode layer 342 and the hydrogen ion sensing layer 324 may form a second interface I2.

Referring to FIG. 5B, because a work function W312 of the first sensing electrode layer 312 is greater than a work function W324 of the hydrogen ion sensing layer 324, a first Schottky junction may be formed between the first sensing electrode layer 312 and the hydrogen ion sensing layer 324. By the first Schottky junction, a first depletion layer with a depth D1 may be formed inside the hydrogen ion sensing layer 324. A first energy barrier height formed by the first Schottky junction is depicted as "Φb1" in FIG. 5B. The first energy barrier height Φb1 may correspond to a difference between the work function W312 of the first sensing electrode layer 312 and electron affinity X322 of the hydrogen ion sensing layer 324.

Similarly, because the work function W342 of the second sensing electrode layer 342 is greater than the work function W324 of the hydrogen ion sensing layer 324, a second Schottky junction may be formed between the second sensing electrode layer 342 and the hydrogen ion sensing layer 324. By the second Schottky junction, a second depletion layer with a depth D2 may be formed inside the hydrogen ion sensing layer 324. The second energy barrier height formed by the second Schottky junction is depicted as "Φb2" in FIG. 5B. Referring to FIG. 5B, the second energy barrier height Φb2 may correspond to a difference between the work function W342 of the second sensing electrode layer 342 and the electron affinity X322 of the hydrogen ion sensing layer 324.

As shown in FIG. 5B, 'Evac' represents a vacuum energy level, 'EF-312' represents a Fermi energy level of the first sensing electrode layer 312, 'EC-324' represents a conduction band energy level of the hydrogen ion sensing layer 324, 'EF-324' represents a Fermi energy level of the hydrogen ion sensing layer 324, 'EV-324' represents a valence band energy level of the hydrogen ion sensing layer 324, and 'EF-342' represents a Fermi energy level of the second sensing electrode layer 342.

Referring to FIG. 5A and FIG. 5B, as an amount of the hydrogen ions flowing into the hydrogen sensing structure 3b is increased, the first energy barrier height Φb1 and the second energy barrier height Φb2 may be increased. As an example, when the hydrogen ions flow into the hydrogen sensing structure 3b in excess of a threshold amount and a reverse bias is applied to the hydrogen sensing structure 3b, leakage current caused by the Schottky barrier may be rapidly decreased as the energy barrier height is increased, compared to when the hydrogen ions do not flow in. The rapid decrease may mean that the leakage current level is decreased by approximately 10² to 10⁶ times. The change in the energy barrier height may be derived by measuring the leakage current. Based on the derived change in the energy barrier height, the concentration of the hydrogen ions flowing into the hydrogen sensing structure 3b can be determined.

According to an embodiment of the present disclosure, as illustrated in FIG. 4B, in the hydrogen sensing structure 3b, the first interface I1 of the first sensing electrode layer 312 and the hydrogen ion sensing layer 324 and the second interface I2 of the second sensing electrode layer 342 and the hydrogen ion sensing layer 324 may be located at different heights from the substrate 101. Accordingly, the concentration of the hydrogen ions flowing into the hydrogen sensing structure 3b can be evaluated at different heights from the substrate 101. Accordingly, concentration distribution of the hydrogen ions inside the semiconductor device 3 can be determined in more detail.

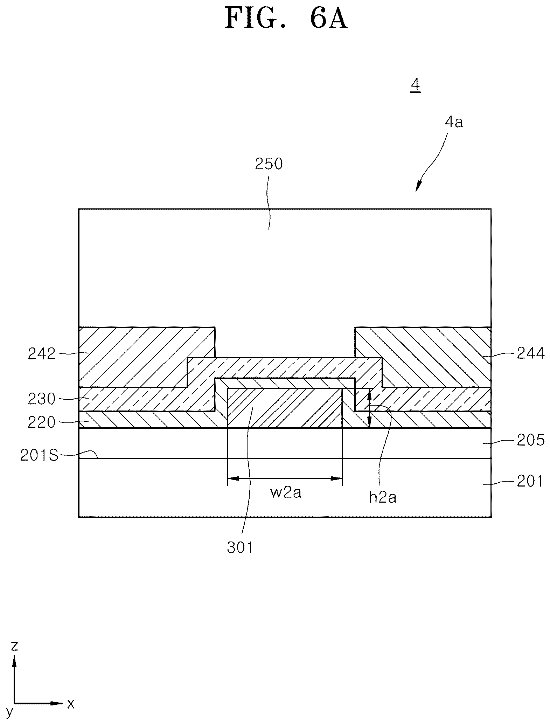

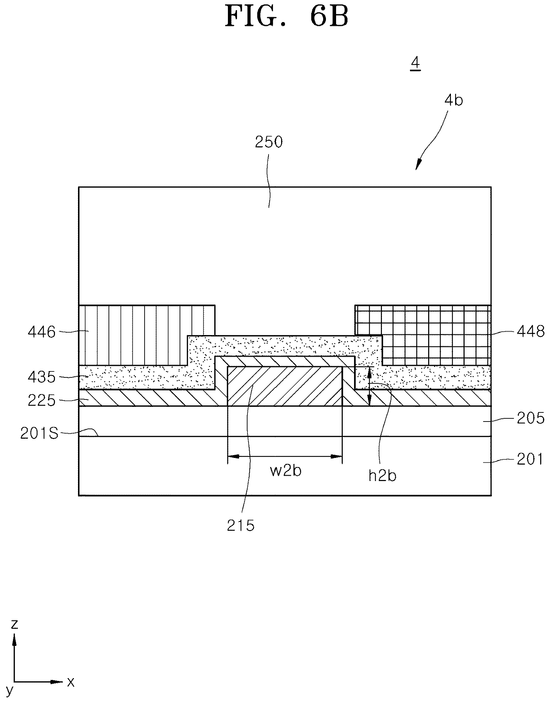

FIG. 6A and FIG. 6B illustrate a semiconductor device 4 according to an embodiment of the present disclosure. Specifically, FIG. 6A illustrates an integrated circuit structure 4a of the semiconductor device 4, and FIG. 6B illustrates a hydrogen sensing structure 4b of the semiconductor device 4.

Referring to FIG. 6A and FIG. 6B, the semiconductor device 4 includes the integrated circuit structure 4a and the hydrogen sensing structure 4b disposed over a substrate 201. The integrated circuit structure 4a and the hydrogen sensing structure 4b are separated from each other in a lateral direction parallel to a surface 201S of the substrate 201. The semiconductor device 4 includes a plurality of cell regions on a substrate 201 and a scribe lane region between the plurality of cell regions. In an embodiment, the integrated circuit structure 4a may be disposed in the plurality of cell regions, and the hydrogen sensing structure 4b may be disposed in a test pattern region located within the scribe lane region. In another embodiment, both the integrated circuit structure 4a and the hydrogen sensing structure 4b may be disposed in the test pattern region.

Referring to FIG. 6B, the integrated circuit structure 4a may include a field effect transistor including an oxide semiconductor layer 230. The integrated circuit structure 4a may be substantially the same as the integrated circuit structure 2a of the semiconductor device 2 described with reference to FIG. 3A.

Referring to FIG. 6B, the hydrogen sensing structure 4b may be disposed separated and spaced apart from the integrated circuit structure 4a of FIG. 6A in a lateral direction, for example, in the x-direction or y-direction. The hydrogen sensing structure 4b may include a hydrogen ion sensing layer 435 disposed over the substrate 201, and a first sensing electrode layer 446 and a second sensing electrode layer 448 disposed on the hydrogen ion sensing layer 435.

The hydrogen ion sensing layer 435 may be disposed on a second buffer layer 225. The material and electrical properties of the hydrogen ion sensing layer 435 may be substantially the same as the material and electrical properties of the hydrogen ion sensing layer 324 described with reference to FIG. 4B. A thickness and profile of the hydrogen ion sensing layer 435 may be substantially the same as the thickness and profile of the oxide semiconductor layer 230 of FIG. 6A.

The first sensing electrode layer 446 and the second sensing electrode layer 448 may be disposed spaced apart from each other in the lateral direction, for example, in the x-direction, on the hydrogen ion sensing layer 435. The hydrogen ion sensing layer 435 may extend in the lateral direction, for example, in the x-direction between the first sensing electrode layer 446 and the second electrode layer 448. The material and electrical properties of each of the first sensing electrode layer 446 and the second electrode layer 449 may be substantially the same as the material and electrical properties of each of the first sensing electrode layers 312 and the second sensing electrode layer 342 of the hydrogen sensing structure 3b described with reference to FIG. 4B. The thickness of each of the first sensing electrode layer 446 and the second sensing electrode layer 448 may be substantially the same as the thickness of each of the source electrode layer 242 and the drain electrode layer 244 of the integrated circuit structure 4a described with reference to FIG. 6A.

Referring to FIG. 6B, an embedding layer 250 may be disposed to cover the hydrogen ion sensing layer 435, the first sensing electrode layer 446, and the second electrode layer 448 over the substrate 201. The passivation layer 250 may isolate the hydrogen ion sensing layer 435, the first sensing electrode layer 446, and the second electrode layer 448 from an external air environment.

An operation method of the hydrogen sensing structure 4b is substantially the same as the operation method of the hydrogen sensing structure 3b of the semiconductor device 3 described with reference to FIG. 5A and FIG. 5B. Based on the change in the electrical resistance properties of the hydrogen ion sensing layer 435 of the hydrogen sensing structure 4b, the concentration of the hydrogen ions flowing into the oxide semiconductor layer 230 of the integrated circuit structure 4a can be effectively determined.

While present disclosure contains many specifics, these should not be construed as limitations on the scope of any invention or of what may be claimed, but rather as descriptions of features that may be specific to particular embodiments of particular inventions. Certain features that are described in the present disclosure in the context of separate embodiments can also be implemented in combination in a single embodiment. Conversely, various features that are described in the context of a single embodiment can also be implemented in multiple embodiments separately or in any suitable sub-combination. Moreover, although features may be described above as acting in certain combinations, one or more features from a combination can in some cases be excised from the combination, and the combination may be directed to a sub-combination or a variation of a sub-combination.

Concepts are disclosed in conjunction with various examples and embodi ments. Those skilled in the art will understand that various modifications, additi ons, and substitutions are possible without departing from the scope and technic al concepts of the present disclosure. The embodiments disclosed in the present specification should be considered from an illustrative standpoint and not consid ered a restrictive standpoint. All changes within the meaning and range of equiv alency of the claims are included within their scope.

## Claims

1. A semiconductor device comprising:
an integrated circuit structure disposed over a substrate; and
a hydrogen sensing structure disposed over the substrate to be laterally spaced apart from the integrated circuit structure,
wherein the integrated circuit structure comprises an oxide semiconductor layer,
wherein the hydrogen sensing structure comprises:
a hydrogen ion sensing layer containing a resistance change material; and
a first sensing electrode layer and a second sensing electrode layer disposed at opposite ends of the hydrogen ion sensing layer, and
wherein a bottom surface and a top surface of the hydrogen ion sensing layer are arranged to have same levels as a bottom surface and a top surface of the oxide semiconductor layer based on the surface of the substrate, respectively.

2. The semiconductor device of claim 1,
wherein an electrical resistance of the hydrogen ion sensing layer is changed depending on concentration of hydrogen ions flowing into the hydrogen ion sensing layer.

3. The semiconductor device of claim 1, wherein the integrated circuit structure comprises a cell transistor and a cell capacitor over the cell transistor, and
the oxide semiconductor layer comprises a channel layer serving as a vertical channel of the cell transistor.

4. The semiconductor device of claim 1, wherein the oxide semiconductor layer comprises at least one selected from indium gallium zinc oxide (IGZO), indium gallium oxide (IGO), and indium zinc oxide (IZO).

5. The semiconductor device of claim 1,
wherein the integrated circuit structure comprises a plurality of conductive layers sequentially stacked over the substrate, and
wherein the oxide semiconductor layer is disposed on a lowermost conductive layer among the plurality of conductive layers.

6. The semiconductor device of claim 1,
wherein the hydrogen ion sensing layer is configured to allow conduction of metal ions through the hydrogen ion sensing layer, and
wherein the first sensing electrode layer is an active electrode layer, and the second sensing electrode layer is an inactive electrode layer.

7. The semiconductor device of claim 6,
wherein the hydrogen ion sensing layer comprises at least one selected from tantalum oxide, aluminum oxide, and lithium phosphate nitride,
wherein the active electrode layer comprises at least one selected from copper (Cu) and lithium (Li), and
wherein the inactive electrode layer comprises at least one selected from platinum (Pt), gold (Au), iridium (Ir), and titanium nitride.

8. The semiconductor device of claim 1,
wherein the hydrogen ion sensing layer comprises oxygen vacancies, and
wherein the first sensing electrode layer is an active electrode layer and the second sensing electrode layer is an inactive electrode layer.

9. The semiconductor device of claim 8,
wherein the hydrogen ion sensing layer comprises at least one selected from hafnium oxide, tantalum oxide, titanium oxide, nickel oxide, zirconium oxide, and aluminum oxide,
wherein the active electrode layer comprises at least one selected from titanium (Ti), tantalum (Ta), aluminum (Al), and hafnium (Hf), and
wherein the inactive electrode layer comprises at least one selected from platinum (Pt), gold (Au), iridium (Ir), and titanium nitride.

10. The semiconductor device of claim 1, wherein the oxide semiconductor layer has a pillar shape.

11. The semiconductor device of claim 1, further comprisingan embedding layer disposed to embed at least the hydrogen ion sensing layer over the substrate.

12. A semiconductor device comprising:
an integrated circuit structure disposed over a substrate; and
a hydrogen sensing structure disposed over the substrate to laterally spaced apart from the integrated circuit structure,
wherein the integrated circuit structure comprises an oxide semiconductor layer,
wherein the hydrogen sensing structure comprises:
a hydrogen ion sensing layer ; and
a first sensing electrode layer and a second sensing electrode layer disposed at opposite ends of the hydrogen ion sensing layer,
wherein each of the first sensing electrode layer and the second sensing electrode layer has a work function greater than a work function of the hydrogen ion sensing layer, and
wherein the hydrogen sensing structure is disposed to have a same level as the integrated circuit structure based on the surface of the substrate.

13. The semiconductor device of claim 12, wherein the first sensing electrode and the second sensing electrode have different work functions.

14. The semiconductor device of claim 12, wherein each of the first sensing electrode and the second sensing electrode has a work function of 5 eV or greater.

15. The semiconductor device of claim 12,
wherein each of the first sensing electrode and the second sensing electrode is configured to form a Schottky junction with the hydrogen ion sensing layer, and
wherein an energy barrier height of the Schottky junction changes depending on an inflow amount of hydrogen ions into the hydrogen sensing structure.

16. The semiconductor device of claim 12,
wherein the hydrogen ion sensing layer comprises at least one selected from gallium-rich indium gallium zinc oxide (Ga-rich IGZO), gallium oxide (Ga₂O₃), and mixed anion zinc oxide (MAZO), and
wherein each of the first sensing electrode layer and the second sensing electrode layer comprises at least one selected from platinum (Pt), palladium (Pd), and silver oxide.

17. The semiconductor device of claim 12, wherein a bottom surface and a top surface of the hydrogen ion sensing layer are arranged to have same levels as a bottom surface and a top surface of the oxide semiconductor layer, respectively.

18. The semiconductor device of claim 12,
wherein the integrated circuit structure comprises a plurality of conductive layers sequentially stacked over the substrate, and
wherein the oxide semiconductor layer is disposed on a lowermost conductive layer among the plurality of conductive layers.

19. The semiconductor device of claim 12, wherein the oxide semiconductor layer has a pillar shape.

20. The semiconductor device of claim 12, further comprising an embedding layer disposed to embed at least the hydrogen ion sensing layer over the substrate.
